# EUROPEAN PATENT APPLICATION

(11) **EP 1 604 977 A1**
(43) Date of publication of application: **14.12.2005**
(21) Application number: 04291373.1
(22) Date of filing: 02.06.2004
(51) Int. Cl.: C07D 207/16, C07D 207/48, A61K 31/401, A61P 25/00

(54) **CIS pyrrolidinyl derivatives and their uses**

(71) Applicant: Faust Pharmaceuticals, 67400 Illkirch (FR)
(72) Inventor: Schann, Stephan, 67118 Geispolsheim (FR); Acher, Francine, 92420 Vaucresson (FR)
(74) Representative: Grosset-Fournier, Chantal Catherine

(57) **Abstract**

The present invention provides new cis pyrrolidinyl of the formula (I) or (II) 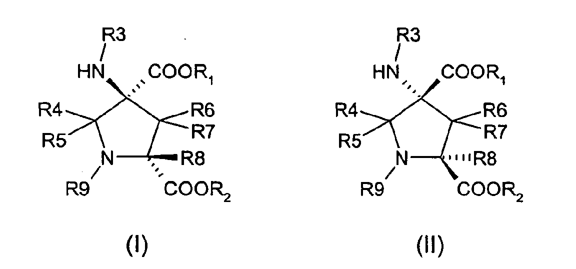 in which:
**R**_{**1**}, **R**_{**2**} or **R**_{**3**} are hydrogen or a carboxy or amino protecting group; **R**_{**4**} to **R**_{**8**} represent hydrogen or an alkyl radical; **R**_{**9**} represents a **(R**_{**10**}**)**_{**n**}**(-R**_{**11**}**)**_{**m**} group wherein **R**_{**10**} is -CO-, -CS-, -O-,-S-, -SO-, -SO₂-, -COO-, -CON(C_{n'}H_{2n'+1})-, -N(C_{n'}H_{2n'+1})CO-, -CSN(C_{n'}H_{2n'+1})- ,-N(C_{n'}H_{2n'+1})CS-, -N(C_{n'}H_{2n'+1})-, -(C_{n'}H_{2n'+1})-, aryl, and **R**_{**11**} is a polar group,
and to their use for the treatment and/or prophylaxis of a condition associated with altered glutamatergic signalling and/or functions, and/or conditions which can be affected by alteration of glutamate level or signalling

## Description

The present invention provides new cis pyrrolidinyl derivatives, pharmaceutical compositions containing them and their use for the treatment and/or prophylaxis of conditions associated with altered glutamatergic signalling and/or functions, and/or conditions which can be affected by alteration of glutamate level or signalling in mammals. This invention further provides new cis pyrrolidinyl derivatives consisting of modulators of nervous system receptors sensitive to glutamate, which makes them particularly suitable for the treatment and/or prophylaxis of acute and chronic neurological and/or psychiatric disorders. In particular embodiments, the new *cis* pyrrolidinyl derivatives of the invention are modulators of metabotropic glutamate receptors (mGluRs). The invention further provides agonists, antagonists or reverse agonists of mGluRs.

Glutamatergic pathways have been shown to be clearly involved in the physiopathology of a number of neuronal damages and injuries. Many nervous system disorders including epilepsy and chronic or acute degenerative processes such as for example Alzheimer's disease, Huntington's disease, Parkinson's disease and amyotrophic lateral sclerosis (Mattson et al., *Neuromolecular Med.,* 65, 2003), but also AIDS-induced dementia, multiple sclerosis, spinal muscular atrophy, retinopathy, stroke, ischemia, hypoxia, hypoglycaemia and various traumatic brain injuries, involve neuronal cell death caused by imbalanced levels of glutamate. It has also been shown that drug-induced neurotoxicity, for example neurotoxic effects of methamphetamine (METH) on striatal dopaminergic neurons, could actually be mediated by over-stimulation of the glutamate receptors (Stephans and Yamamoto, 1994, Synapse, 17, 203-209). Antidepressant and anxiolytic-like effects of compounds acting on glutamate have also been observed on mice, suggesting that glutamatergic transmission is implicated in the pathophysiology of affective disorders such as major depression and anxiety (Cryan et al., 2003, Eur. J. Neurosc., 17(11):2409-17). Consequently, any compound able to modulate glutamatergic signalling of function would constitute a promising therapeutic compound for many disorders of the nervous system.

Moreover, compounds modulating glutamate level or signalling may be of great therapeutic value for diseases and/or disorders not directly mediated by glutamate levels and/or glutamate receptors malfunctioning, but which could be affected by alteration of glutamate levels or signalling.

In the central nervous system (CNS), L-glutamate (Glu) is the main excitatory neurotransmitter and is referred to as an excitatory amino-acid (EAA), and gamma-aminobutyric acid (GABA) is the main inhibitory neurotransmitter. The balance between excitation and inhibition is of utmost importance to CNS functions, and dysfunctions of either of the two can be related to various neurological disorders.

Glutamate is ubiquitously distributed in the nervous system in high concentrations, especially in the brain and spinal cord of mammals, where it is working at a variety of excitatory synapses being thereby involved in virtually all physiological functions such as motor control, vision, central control of heart, processes of learning and memory. However, a large number of studies have established that cellular communication involving glutamate can also lead to a mechanism of cell destruction. This combination of neuroexcitatory activities and neurotoxic properties is called excitotoxicity.

Glutamate operates through two classes of receptors (Bräuner-Osborne et al., *Journal of Medicinal Chemistry,* 2609, 2000). The first class of glutamate receptors is directly coupled to the opening of cation channels in the cellular membrane of the neurons. Therefore they are called ionotropic glutamate receptors (IGluRs). The IGluRs are divided in three subtypes, which are named according to the depolarizing action of their selective agonists: N-methyl-D-aspartate (NMDA), α-amino-3-hydroxy-5-methylisoxazole-4-propionic acid (AMPA), and kainic acid (KA). The second class of glutamate receptor consists of G-protein coupled receptors (GPCRs) called metabotropic glutamate receptors (mGluRs). These mGluRs are localized both pre- and post-synaptically. They are coupled to multiple second messenger systems and their role is to regulate the activity of the ionic channels or enzymes producing second messengers via G-proteins binding the GTP (Conn and Pin, *Annu . Rev. Pharmacol. Toxicol*., 205, 1997). Although they are generally not directly involved in rapid synaptic transmission, the mGluRs modulate the efficacy of the synapses by regulating either the post-synaptic channels and their receptors, or the pre-synaptic release or recapture of glutamate. Therefore, mGluRs play an important role in a variety of physiological processes such as long-term potentiation and long-term depression of synaptic transmission, regulation of baroreceptive reflexes, spatial learning, motor learning, and postural and kinetic integration.

To date, eight mGluRs have been cloned and classified in three groups according to their sequence homologies, pharmacological properties and signal transduction mechanisms. Group I is constituted of mGluR1 and mGluR5, group II of mGluR2 and mGluR3 and group III of mGluR4, mGluR6, mGluR7 and mGluR8 (Pin and Acher, *Current Drug Targets - CNS & Neurological Disorders,* 297, 2002; Schoepp et al., *Neuropharmacology,* 1431, 1999).

Numerous studies have already described the potential applications of mGluRs modulators in neuroprotection. Most of them are directed to group I and II mGluRs (see Bruno et al., *J. Cereb. Blood Flow Metab.,* 1013, 2001 for review). For instance, antagonist compounds of group I mGluRs showed interesting results in animal models for anxiety and postischemic neuronal injury (Pilc et al., *Neuropharmacology,* 181, 2002; Meli et al., *Pharmacol. Biochem. Behav.,* 439, 2002), agonists of group II mGluRs showed good results in animal models for Parkinson and anxiety (Konieczny et al., *Naunyn-Schmiederbergs Arch. Pharmacol*., 500, 1998; Schoepp et al., *CNS Drug Reviews,* 1, 1999) and more recently, agonists of group III mGluRs showed promising results in animal models for Parkinson and neurodegeneration (Marino et al., *PNAS*, 13668, 2003; Bruno et al., *Neuropharmacology,* 2223, 2000).

mGluRs modulators can be classified in two families depending on their site of interaction with the receptor (see Bräuner-Osborne et al., *Journal of Medicinal Chemistry,* 2609, 2000 for review). The first family consists in orthosteric modulators (or competitive modulators) able to interact with the active site of the mGluRs, which is localized in the large extra-cellular N-terminal part of the receptor (about 560 amino acids). Therefore, they are considered as glutamate analogs and constitute a highly polar family of ligand. Examples of orthosteric modulators are S-DHPG or LY-367385 for group I mGluRs, LY-354740 or (2R-4R)-APDC for group II mGluRs and ACPT-I or L-AP4 for group III mGluRs. These ligands have the advantage of being selective for mGluRs due to their amino-acid structures and therefore have a reduced potential for side effects. The second family of mGluRs modulators consists in allosteric modulators that interact with a different site from the extracellular active site of the receptor. Their action results in a modulation of the effects induced by the endogenous ligand glutamate. Examples of these allosteric modulators are Ro 67-7476, MPEP or SIB-1893 for group I mGluRs (Knoflach, et al., *PNAS*, 13402, 2001; Gasparini et al., *Current Opinion* in *Pharmacology,* 43, 2002), LY181837 or LY487379 for group II mGluRs (Johnson et al., *Journal of Medicinal Chemistry,* 3189, 2003) and PHCCC, MPEP or SIB-1893 for group III mGluRs (Marino et al., *PNAS*, 13668, 2003; Maj et al., *Neuropharmacology,* 895, 2003; Mathiesen et al., *British Journal of Pharmacology,* 1026, 2003). The main advantage of these ligands is their high lipid solubility compared to orthosteric modulators, enhancing their ability to cross the blood brain barrier. One of their possible disadvantages is their weaker selectivity for mGluRs because of structures that are often common to many GPCRs. Consequently, the use of allosteric modulators as drugs may lead to various undesirable side effects.

The present invention provides new cis pyrrolidinyl derivatives acting as modulators of the mGluRs and especially as orthosteric modulators of the mGluRs. Up to now, only one subtype selective competitive agonist was described (namely (S)-3,4-DCPG for mGluR8 ; Thomas, et al., *Neuropharmacology, 1223,* 1998). As above-mentioned, this family of ligands is highly polar and derived from the L-Glutamate structure. It is the case of LY354740, a high affinity mGluR2 and mGluR3 agonist, (2R-4R)-APDC, a mGluR2 and mGluR3 agonist, (S)-PPG, L-AP4, L-SOP, (S)-3,4-DCPG, ACPT-I all agonists of group III mGluRs. The structures of these compounds are as follows:

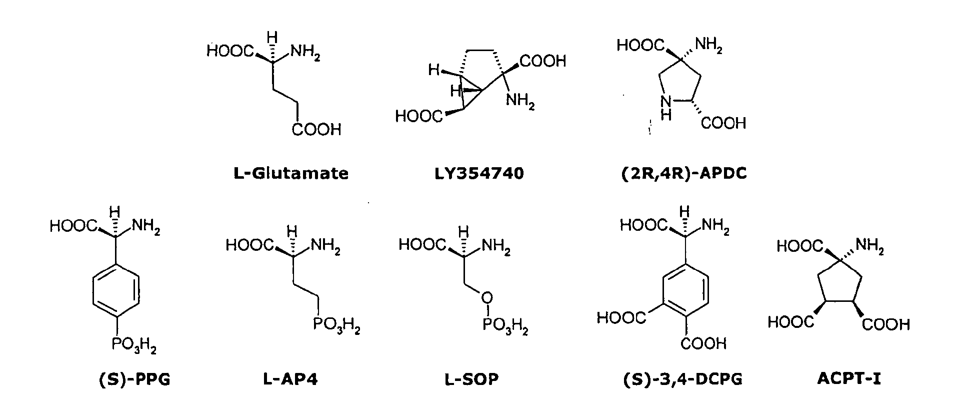

The APDCs were first described in the mid 90's (Tanaka et al., *Tetrahedron: Asymmetry,* 1641, 1995; Monn et al., *Journal of Medicinal Chemistry,* 2990, 1996) displaying an *affinity for* different mGluRs. Examples of APDC derivatives are APDCs with no substitution on the pyrrolidinyl nitrogen as mGluRs agonists (US 5,473,077), and APDC derivatives with a trans configuration for the two -COOH groups in positions 2 and 4 as mGluRs antagonists (EP 0 703 218).

Different APDCs derivatives displaying a substitution on the pyrrolidinyl nitrogen have already been studied by Tückmantel et al. (*Bioorganic & Medicinal Chemistry Letters,* 601, 1997) who described N-benzyl-(2R-4R)-APDC, Valli et al., (*Bioorganic & Medicinal Chemistry Letters,* 1985, 1998) who described trans (2R-4R)-APDC derivatives with bulky substitutions on the pyrrolidinyl nitrogen as mGluR antagonists, Kozikowski et al., (*Bioorganic & Medicinal Chemistry Letters,* 1721, 1999) who described N-amino- (2R-4R) -APDC as a mGluR partial agonist and Mukhopadhyaya et al., (*Bioorganic & Medicinal Chemistry Letters,* 1919, 2001) who described N-substituted derivatives of the *trans* (2R-4R)-APDC as mGluRs modulators.

In comparison with modulators of the other groups of mGluRs, all the known orthosteric modulators binding to group III mGluRs have an additional distal polar group. The "distal polar group" means the polar moiety corresponding to the -CH₂-CH₂-COOH of glutamate. Examples of such modulators are PPG, L-AP4, L-SOP, (3,4)-DCPG and ACPT-I. This excess in polar properties seems to be necessary for a good binding to group III mGluRs.

In view of the foregoing, the inventors have studied new derivatives of APDC having both -COOH groups in positions 2 and 4 in a cis configuration and substituted on the pyrrolidinyl nitrogen with an additional polar function spaced out through a spacer group. These derivatives are named cis-derivatives in comparison with trans-derivatives having both -COOH groups in positions 2 and 4 in a trans configuration. Compared to ACPTs, the compounds of the invention display the following advantages: a reduced number of asymmetric carbons and an easier way of synthesis.

The present invention relates to new cis pyrrolidinyl derivatives, pharmaceutical compositions containing them and their use for the treatment and/or prophylaxis of conditions associated with altered glutamatergic signalling and/or functions, and/or conditions which can be affected by alteration of glutamate level or signalling in mammals. It further relates to a method of treating and/or preventing conditions associated with altered glutamatergic signalling and/or functions, and/or conditions which can be affected by alteration of glutamate level or signalling in a mammal. In further embodiments the new cis pyrrolidinyl derivatives are modulators of mGluRs of the nervous system. In preferred embodiments the compounds of the invention are agonists, antagonists or reverse agonists of the mGluRs. The conditions associated with altered glutamatergic signalling and/or functions, and/or conditions which can be affected by alteration of glutamate level or signalling are epilepsy, including newborn, infantile, childhood and adult syndromes, partial (localization-related) and generalized epilepsies, with partial and generalized, convulsive and non-convulsive seizures, with and without impairment of consciousness, and status epilepticus; Dementias, including dementias of the Alzheimer's type (DAT), Pick's disease, vascular dementias, Lewy-body disease, dementias due to metabolic, toxic and deficiency diseases (including alcoholism, hypothyroidism, and vitamin B12 deficiency), AIDS-dementia complex, Creutzfeld-Jacob disease and atypical subacute spongiform encephalopathy; Parkinsonism and movement disorders, including multiple system atrophy, progressive supranuclear palsy, hepatolenticular degeneration, chorea (including Huntington's disease and hemiballismus), athetosis, dystonias (including spasmodic torticollis, occupational movement disorder, Gilles de la Tourette syndrome), tardive or drug induced dyskinesias, tremor and myoclonus; Motor neuron disease or amyotrophic lateral sclerosis (ALS); Other neurodegenerative and/or hereditary disorders of the nervous system, including spinocerebrellar degenerations such as Friedrich's ataxia and other hereditary cerebellar ataxias, predominantly spinal muscular atrophies, hereditary neuropathies, and phakomatoses; Disorders of the peripheral nervous system, including trigeminal neuralgia, facial nerve disorders, disorders of the other cranial nerves, nerve root and plexus disorders, mononeuritis such as carpal tunnel syndrome and sciatica, hereditary and idiopathic peripheral neuropathies, inflammatory and toxic neuropathies; Multiple sclerosis and other demyelinating diseases of the nervous system; Infantile cerebral palsy (spastic), monoplegic, paraplegic or tetraplegic; Hemiplegia and hemiparesis, flaccid or spastic, and other paralytic syndromes; Cerebrovascular disorders, including subarachnoid hemorrhage, intracerebral hemorrhage, occlusion and stenosis of precerebral arteries, occlusion of cerebral arteries including thrombosis and embolism, brain ischemia, stroke, transient ischemic attacks, atherosclerosis, cerebrovascular dementias, aneurysms, cerebral deficits due to cardiac bypass surgery and grafting; Migraine, including classical migraine and variants such as cluster headache; Headache; Myoneural disorders including myasthenia gravis, acute muscle spasms, myopathies including muscular dystrophies, mytotonias and familial periodic paralysis; Disorders of the eye and visual pathways , including retinal disorders, and visual disturbances,; Intracranial trauma/injury and their sequels; Trauma/injury to nerves and spinal cord and their sequels; Poisoning and toxic effects of nonmedicinal substances; Accidental poisoning by drugs, medicinal substances and biologicals acting on the central, peripheral and autonomic system; Neurological and psychiatric adverse effects of drugs, medicinal and biological substances; Disturbance of sphincter control and sexual function; Mental disorders usually diagnosed in infancy, childhood or adolescence, including : mental retardation, learning disorders, motor skill disorders, communication disorders, pervasive developmental disorders, attention deficit and disruptive behaviour disorders, feeding and eating disorders, TIC disorders, elimination disorders; Delirium and other cognitive disorders; Substance related disorders including: alcohol-related disorders, nicotine-related disorders, disorders related to cocaine, opioids, cannabis, hallucinogens and other drugs; Schizophrenia and other psychotic disorders; Mood disorders, including depressive disorders and bipolar disorders; Anxiety disorders, including panic disorders, phobias, obsessive-compulsive disorders, stress disorders, generalized anxiety disorders; Eating disorders, including anorexia and bulimia; Sleep disorders, including dyssomnias (insomnia, hypersomnia, narcolepsy, breathing related sleep disorder) and parasomnias; Medication-induced movement disorders (including neuroleptic-induced parkinsonism and tardive dyskinesia); Endocrine and metabolic diseases including diabetes, disorders of the endocrine glands, hypoglycaemia; Acute and chronic pain; Nausea and vomiting; Irritable bowel syndrome.

First, the present invention concerns compounds of the general formula (I) or (II): 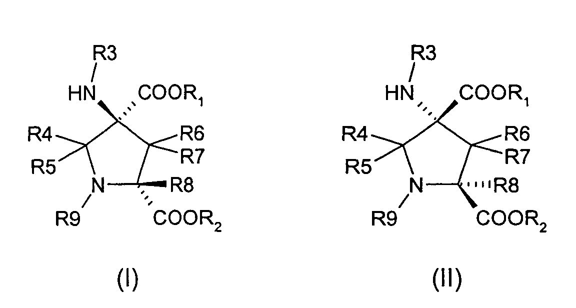 in which
**R**_{**1**} and **R**_{**2**} are each individually hydrogen or a carboxy-protecting group;
**R**_{**3**} is hydrogen or an amino-protecting group;
**R**_{**4**} to **R**_{**8**}**,** identical to or different from each other, represent a hydrogen atom, a halogen atom, an alkyl radical or an aryl radical, a -OH or a -SH, these radicals themselves being substituted where appropriate;
**R**_{**4**} and **R**_{**5**} can form a carbonyl bond or a thiocarbonyl bond;
**R**_{**9**} represents a **(R**_{**10**}**)**_{**n**}**(-R**_{**11**}**)**_{**m**} group wherein
   **n** represents an integrer of from 0 to 4;
   **m** represents an integrer of from 1 to 3;
   **R**_{**10**} is a moiety selected in the group consisting of:
   (i) CH₂
   (ii) 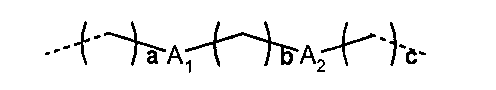
   (iii) 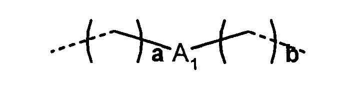 with:
      **a, b** and c are, independently from one another, an integer ranging from 0 to 4 ;
      **A**_{**1**} and **A**_{**2**} are, independently from one another, a moiety selected in the group consisting of -CO-, -CS-, -O-, -S-, -SO-, -SO₂-, -COO-, -CON(C_{n'}H_{2n'+1})-, -N(C_{n'}H_{2n'+1})CO-, -CSN(C_{n'}H_{2n'+1})-, -N(C_{n'}H_{2n'}+1)CS-, -N(C_{n'}H_{2n'+1})-, -(C_{n'}H_{2n'+1})-, aryl, cycloalkyl, -1,4-piperidinyl, 1,4-piperazinyl,
      with **-(C**_{**n'**}**H**_{**2n'+1**}**)-** designating a straight or branched chain, or cyclic carbon radical, or combination thereof, which may be fully saturated, mono or polyunsaturated and can include di- and multi-moieties, and having from 1 to 8 carbon atoms,
      with **n'** is, independently from one another, an integer ranging from 1 to 8, preferably from 1 to 4, preferably from 1 to 3 and more preferably from 1 to 2,
   **R**_{**11**} is a polar group such as -COOH, -SO₃H, -SO₂H, -PO₃H₂, -PO₂H, -B(OH)₂, tetrazol, -CO(C_{m'}H_{2m'+1}), -C(NOH)(C_{n'}H_{2m'+1}), -CS(C_{m'}H_{2m'+1}), -OH,-0(C_{m'}H_{2m'+1}), -OCO(C_{m'}H_{2m'+1}), -SH, -S(C_{m'}H_{2m'+1}), -SCO(C_{m'}H_{2m'+1}), -NH₂, -NHOH, -N(C_{m'}H_{2m'+1})₂, -N⁺(C_{m'}H_{2m'+1})₃, -NHCO(C_{m'}H_{2m'+1}), -NHSO₂(C_{m'}H_{2m'+1})₂, -NHCON(C_{m'}H_{2m'+1}), -NHCSN(C_{m'}H_{2m'+1}), -CON(C_{m'}H_{2m'+1})₂, -CSN(C_{m'}H_{2m'+1})₂, -SO₂N(C_{m'}H_{2m'+1})₂;
   with **m'** is, independently from one another, an integer ranging from 0 to 4,
as well as their pharmaceutically acceptable salts, or metabolically labile esters or amides.

The present invention also includes all physical forms of the compounds of formulae (I) and (II), including crystalline solvates.

Within the scope of the present invention, the term "patient" designates human subjects or other mammals.

The compounds of formulae (I) and (II) are modulators of mGluRs functions, and are especially agonists, antagonists or reverse agonists of mGluRs functions.

Particularly preferred compounds of formulae (I) and (II) are the following compounds of formulae (Ia) and (IIa) : 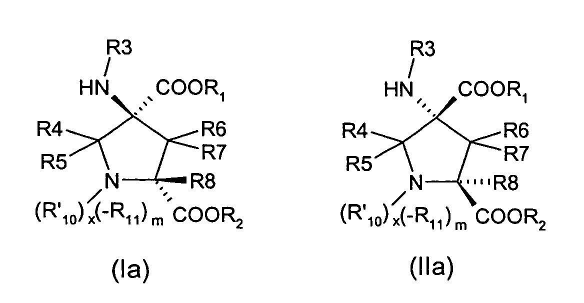 in which:
**R**_{**1**} to **R**_{**8**}**, R**_{**11**}**,** and **m** are as defined for formulae (I) and (II) above;
**X** represents an integer from 1 to 4**;**
**R'**_{**10**} is a moiety selected in the group consisting of:
   (i) 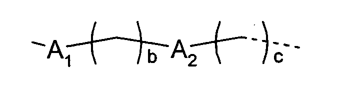
   (ii) 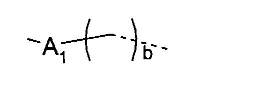 with:
      **b, c, A**_{**1**} and **A**_{**2**} being as defined in (I) and (II) above.

Preferred compounds of the invention are compounds of formulae (Ia) and/or (IIa) where **A**_{**1**} is a moiety selected in the group consisting of -CO-, -CS-, or -SO₂.

In another aspect, preferred compounds of the invention are compounds of formulae (Ia) and/or (IIa) where **A**_{**2**} is a moiety selected in the group consisting of aryl, -NH-, or -(C_{n'}H_{2n'+1})- as defined above.

The invention relates more particularly to compounds as defined above, of the formula (Ib) or (IIb): 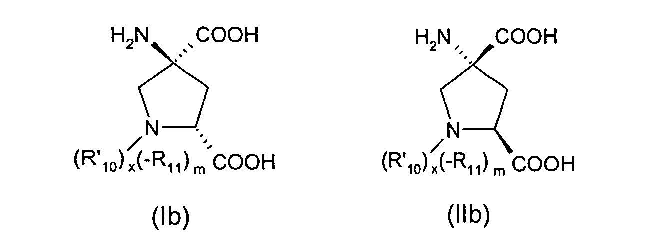 in which **R'**_{**10**}**, R**_{**11**}**, x** and **m** are as defined above.

The invention concerns more particularly compounds as defined above, of the formula (I), (Ia), (Ib),(II), (IIa), or (IIb) wherein **R**_{**11**} is an acidic group such as -COOH, -SO₃H, -SO₂H, -PO₃H₂, -PO₂H, -B(OH)₂, and tetrazol.

Preferred compounds of the formula (Ib), (IIb) wherein **R**_{**11**} is an acidic group as defined above, are listed below:
(2S,4R)-4-Amino-pyrrolidine-1,2,4-tricarboxylic acid of formula : 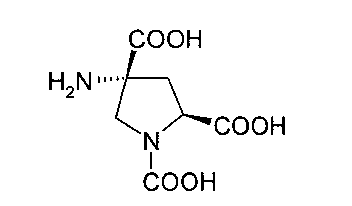
(2R,4S)-4-Amino-pyrrolidine-1,2,4-tricarboxylic acid of formula : 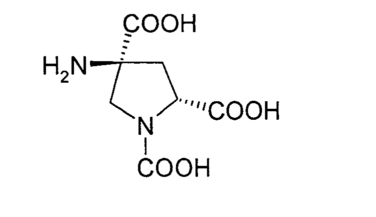
(2S,4R)-4-Amino-1-oxalyl-pyrrolidine-2,4-dicarboxylic acid of formula : 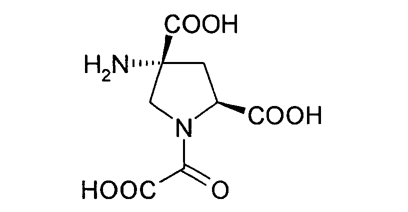
(2R,4S)-4-Amino-1-oxalyl-pyrrolidine-2,4-dicarboxylic acid of formula : 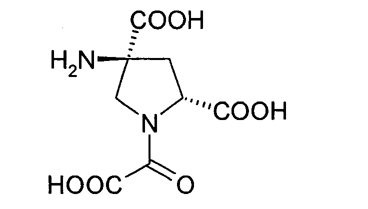
(2S,4R)-4-Amino-1-(2-carboxy-acetyl)-pyrrolidine-2,4-dicarboxylic acid of formula : 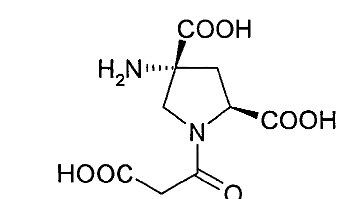
(2R,4S)-4-Amino-1-(2-carboxy-acetyl)-pyrrolidine-2,4-dicarboxylic acid of formula : 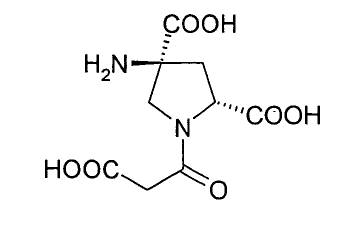
(2S,4R)-4-Amino-1-(3-carboxy-propionyl)-pyrrolidine-2,4-dicarboxylic acid of formula : 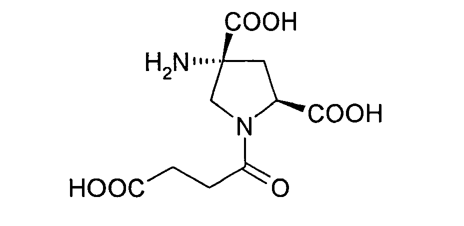
(2R,4S)-4-Amino-1-(3-carboxy-propionyl)-pyrrolidine-2,4-dicarboxylic acid of formula : 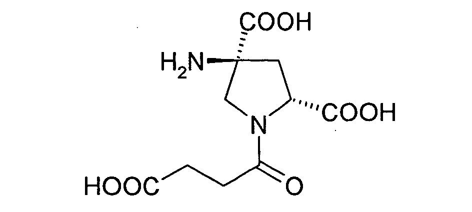
(2S,4R)-4-Amino-1-(4-carboxy-butyryl)-pyrrolidine-2,4-dicarboxylic acid of formula : 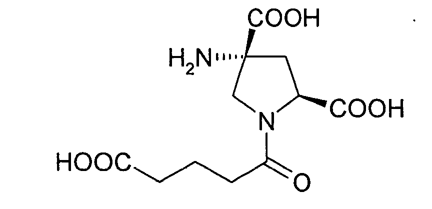
(2R,4S)-4-Amino-1-(4-carboxy-butyryl)-pyrrolidine-2,4-dicarboxylic acid of formula : 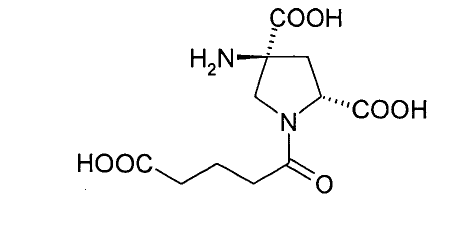
(2S,4R)-4-Amino-1-(5-carboxy-pentanoyl)-pyrrolidine-2,4-dicarboxylic acid of formula : 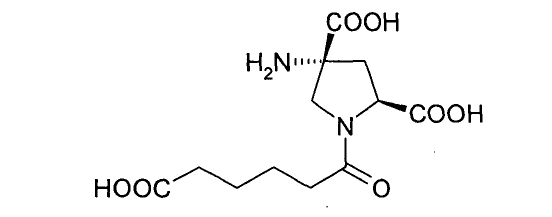
(2R,4S)-4-Amino-1-(5-carboxy-pentanoyl)-pyrrolidine-2,4-dicarboxylic acid of formula : 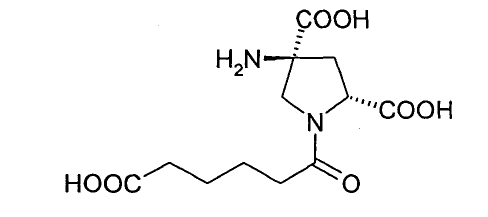
(2S,4R)-4-Amino-1-((E)-3-carboxy-acryloyl)-pyrrolidine-2,4-dicarboxylic acid of formula : 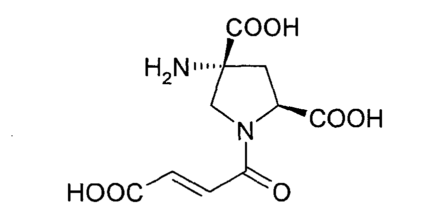
(2R,4S)-4-Amino-1-((E)-3-carboxy-acryloyl)-pyrrolidine-2,4-dicarboxylic acid of formula : 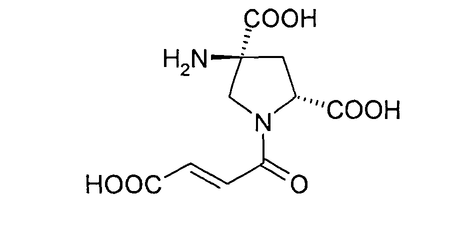
(2S,4R)-4-Amino-1-((Z)-3-carboxy-acryloyl)-pyrrolidine-2,4-dicarboxylic acid of formula : 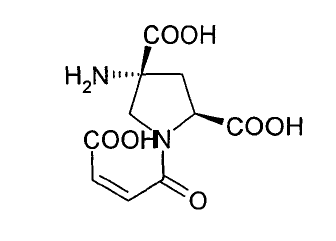
(2R,4S)-4-Amino-1-((Z)-3-carboxy-acryloyl)-pyrrolidine-2,4-dicarboxylic acid of formula : 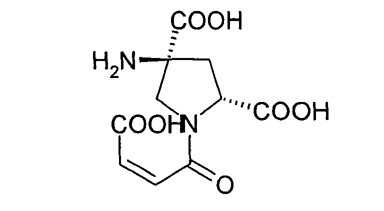
(2S,4R)-4-Amino-1-(2-carboxy-benzoyl)-pyrrolidine-2,4-dicarboxylic acid of formula : 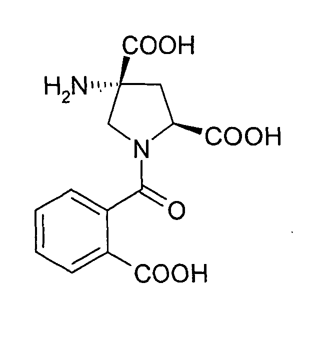
(2R,4S)-4-Amino-1-(2-carboxy-benzoyl)-pyrrolidine-2,4-dicarboxylic acid of formula : 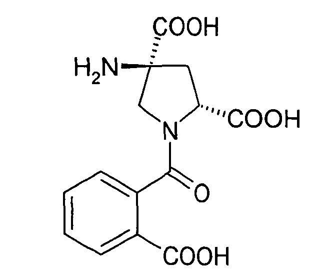
(2S,4R)-4-Amino-1-(3-carboxy-benzoyl)-pyrrolidine-2,4-dicarboxylic acid of formula : 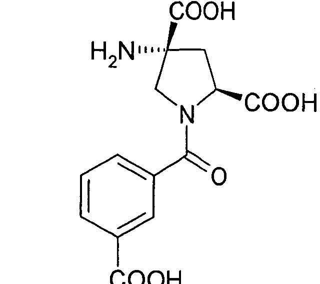
(2R, 4S) -4-Amino-1- (3-carboxy-benzoyl) -pyrrolidine-2, 4-dicarboxylic acid of formula : 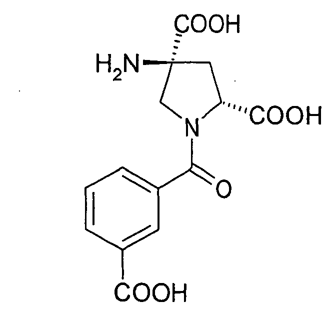
(2S,4R)-4-Amino-1-(4-carboxy-benzoyl)-pyrrolidine-2,4-dicarboxylic acid of formula : 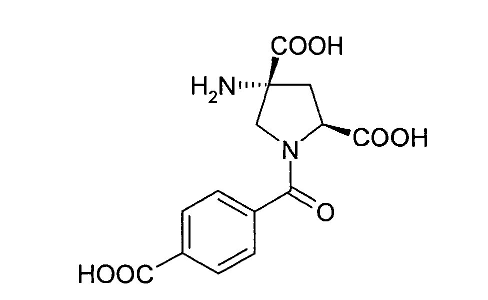
(2R,4S)-4-Amino-1-(4-carboxy-benzoyl)-pyrrolidine-2,4-dicarboxylic acid of formula : 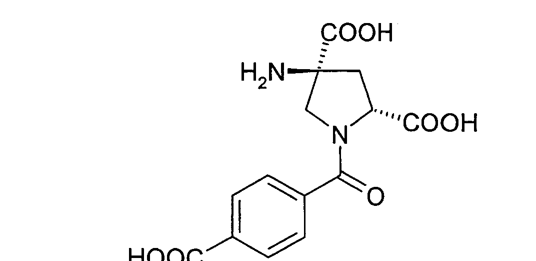
(2S,4R)-4-Amino-1-(2-carboxy-benzenesulfonyl)-pyrrolidine-2,4-dicarboxylic acid of formula : 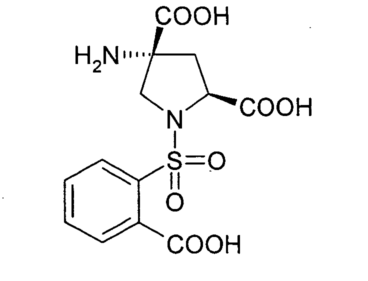
(2R,4S)-4-Amino-1-(2-carboxy-benzenesulfonyl)-pyrrolidine-2,4-dicarboxylic acid of formula : 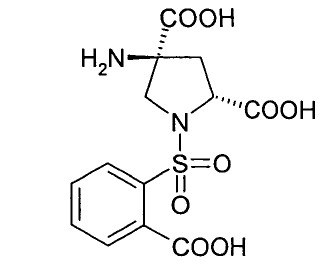
(2S,4R)-4-Amino-1-(3-carboxy-benzenesulfonyl)-pyrrolidine-2,4-dicarboxylic acid of formula : 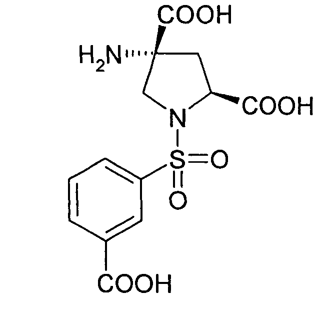
(2R,4S)-4-Amino-1-(3-carboxy-benzenesulfonyl)-pyrrolidine-2,4-dicarboxylic acid of formula : 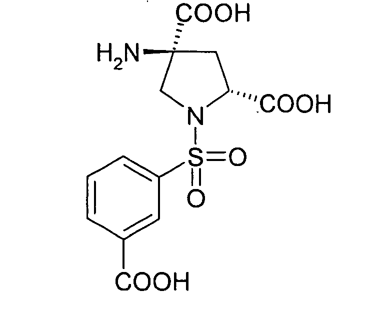
(2S,4R)-4-Amino-1-(4-carboxy-benzenesulfonyl)-pyrrolidine-2,4-dicarboxylic acid of formula : 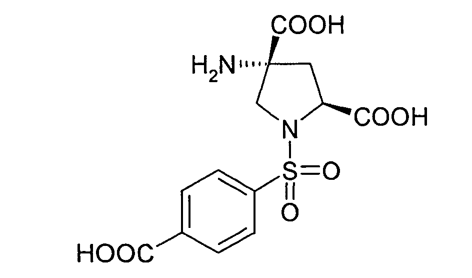
(2R,4S)-4-Amino-1-(4-carboxy-benzenesulfonyl)-pyrrolidine-2,4-dicarboxylic acid of formula : 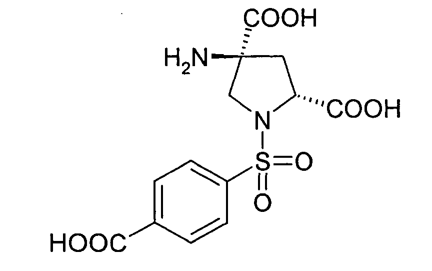
(2S,4R)-4-Amino-1-(3-carboxy-propylcarbamoyl)-pyrrolidine-2,4-dicarboxylic acid of formula : 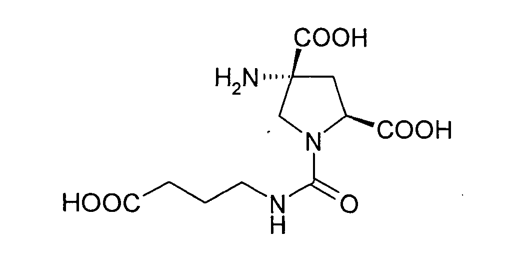
(2R,4S)-4-Amino-1-(3-carboxy-propylcarbamoyl)-pyrrolidine-2,4-dicarboxylic acid of formula : 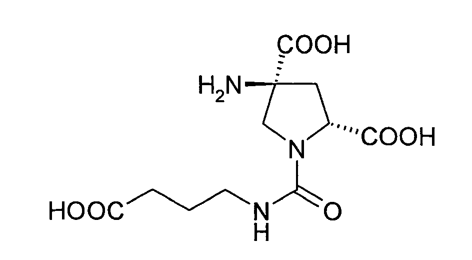
(2S,4R)-4-Amino-1-(3-carboxy-propylthiocarbamoyl)-pyrrolidine-2,4-dicarboxylic acid of formula : 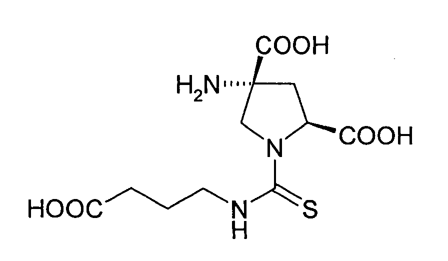
(2R,4S)-4-Amino-1-(3-carboxy-propylthiocarbamoyl)-pyrrolidine-2,4-dicarboxylic acid of formula :

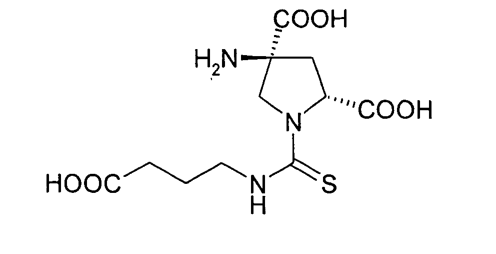

The invention concerns more particularly compounds as defined above of the formula (I), (Ia), (Ib), (II), (IIa), or (IIb) wherein **R**_{**11**} is a group selected from -CO(C_{m'}H_{2m'+1}), -C(NOH)(C_{m'}H_{2m'+1}), -CS(C_{m'}H_{2m'+1}), -OH, -O(C_{m'}H_{2m'+1}), -OCO(C_{m'}H_{2m'+1}), -SH, -S(C_{m'}H_{2m'+1)}, -SCO(C_{m'}H_{2m'+1}), -NH₂, -NHOH, -N(C_{m'}H_{2m'+1})₂, -N⁺(C_{m'}H_{2m'+1})₃, -NHCO(C_{m'}H_{2m'+1}), -NHSO₂(C_{m'}H_{2m'+1})₂, -NHCON(C_{m'}H_{2m'+1}), -NHCSN(C_{m'}H_{2m'+1}), -CON(C_{m'}H_{2m'+1})₂, -CSN(C_{m'}H_{2m'+1})₂, -SO₂N(C_{m'}H_{2m'+1})₂; with m' being as defined above.

Preferred compounds of the formula (Ib), (IIb) wherein **R**_{**11**} is as mentioned above, correspond to :
(2S,4R)-4-Amino-1-(2-hydroxy-acetyl)-pyrrolidine-2,4-dicarboxylic acid of formula : 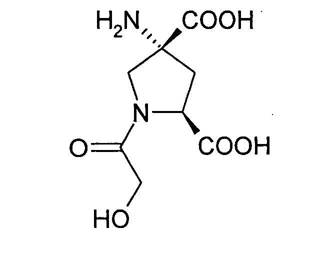
(2R,4S)-4-Amino-1-(2-hydroxy-acetyl)-pyrrolidine-2,4-dicarboxylic acid of formula : 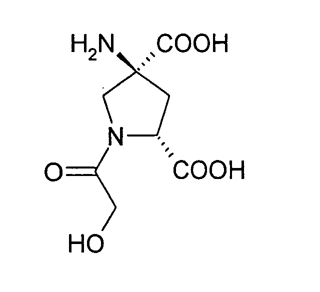
(2S,4R)-4-Amino-1-(2-methoxy-acetyl)-pyrrolidine-2,4-dicarboxylic acid of formula : 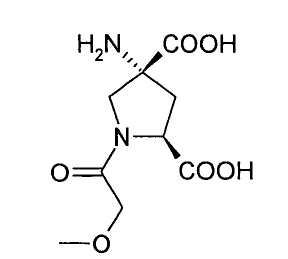
(2R,4S)-4-Amino-1-(2-methoxy-acetyl)-pyrrolidine-2,4-dicarboxylic acid of formula : 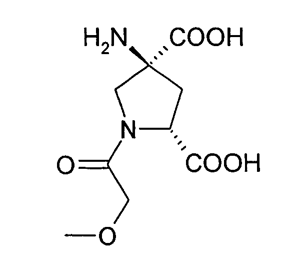
(2S,4R)-1-(2-Acetylamino-acetyl)-pyrrolidine-2,4-dicarboxylic acid of formula : 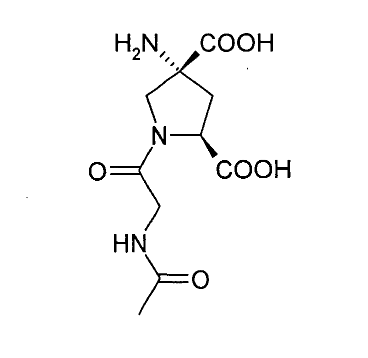
(2R,4S)-1-(2-Acetylamino-acetyl)-pyrrolidine-2,4-dicarboxylic acid of formula :

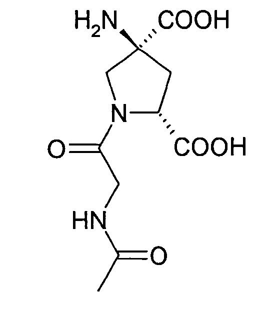

Unless specified otherwise, the term "aryl" means the residue of a 5 or 6 membered aromatic or heteroaromatic ring, the residue of a bicyclic aromatic or heteroaromatic ring; these residues can be further substituted. The ring "aryl" optionally comprises one to three heteroatoms selected from N, O and S.

The term "C_{n'}H_{2n'+1}" as used herein, alone or in combination, is intended to designate a straight or branched chain, or cyclic carbon radical, or combination thereof, which may be fully saturated, mono- or polyunsaturated and can include di- and multi-moieties. Typically, an C_{n'}H_{2n'+1} moiety will have from 1 to 8 carbon atoms, with those moieties having 4 or fewer carbon atoms being preferred in the present invention. In rather preferred embodiment, the C_{n'}H_{2n'+1} moieties of the invention are alkyl.

As used herein, the term "alkyl" is a shorter C_{n'}H_{2n'+1} chain having eight or fewer carbon atoms (e.g. n'≤ 8), preferably six or fewer carbon atoms (e.g. n'≤ 6), and even more preferably 4 or fewer carbon atoms (i.e. C₁₋₄). Typically, a C₁₋₄ alkyl moiety according to the invention will have from 1 to 2 carbon atoms. Examples of saturated alkyl moieties include, but are not limited to, methyl, ethyl, n-propyl, isopropyl, n-butyl, t-butyl, isobutyl, sec-butyl, tert-butyl, cyclopropyl, cyclobutyl, cyclopentyl, cyclohexyl, cycloheptyl and cyclooctyl, (cyclohexyl)methyl, cyclopropylmethyl, n-pentyl, isopentyl, n-hexyl, isohexyl, n-heptyl, isoheptyl, n-octyl, and the like. An unsaturated alkyl moiety is one comprising one or more double bonds or triple bonds. Examples of unsaturated alkyl moieties include, but are not limited to, aromatic cycles such as phenyl and benzyl. Additionally, the term "alkyl" is intended to further include those derivatives of alkyl comprising at least one heteroatom, selected from the group consisting of O, N and/or S (i.e. at least one carbon atom is replaced with one heteroatom). These alkyl derivatives are widely named "heteroalkyl" and as alkyl above described are intended to designate, by themselves or as part of another substituent, stable straight or branched chains, or cyclic moieties, or combinations thereof. According to specific embodiment, the nitrogen and sulfur atoms when present in the said heteroalkyl are further oxidized and/or the nitrogen heteroatom is quaternized. The heteroatom may be placed at any position of the heteroalkyl moiety, including the position at which the alkyl moiety is attached to the remainder of the molecule.

The terms "cycloalkyl" and "heterocycloalkyl", by themselves or as part of another substituent, are intended to designate cyclic versions of the above "alkyl" and "heteroalkyl", respectively. They include bicyclic, tricyclic and polycyclic versions thereof.

The terms "carboxy-protecting group" and "amino-protecting group" are employed in order to reversibly preserve a reactively susceptible amino or carboxy functionality while reacting other functional groups on the compound. Examples of amino-protecting groups are t-butoxycarbonyl (t-Boc), allyloxycarbonyl and benzyloxycarbonyl (CbZ). Further examples of these groups are found in E. Haslam (Protective groups in organic chemistry, J.G.W. McOmie, 1973, chapter 2) and P.G.M. Wutz (Protective groups in organic synthesis, 1991, chapter 5). Examples of carboxy-protecting groups are allyl, benzyl and t-butyl. Further examples of these groups are found in E. Haslam (Protective groups in organic chemistry, J.G.W. McOmie, 1973, chapter 5) and T.W. Greene and P.G.M. Wutz (Protective groups in organic synthesis, 1991, chapter 5).

The following general method may be used in producing the compounds of the invention. This method of synthesis is based on the work of Monn et al. (Journal of Medicinal Chemistry, 39(15): 2990-3000, 1996) directed to the synthesis of APDCs (4-aminopyrrolidine-2,4-dicarboxylates).

The schemes below illustrate the general process used to synthesize the intermediate **6A** which serves as the backbone for the synthesis of compounds corresponding to formula (II): 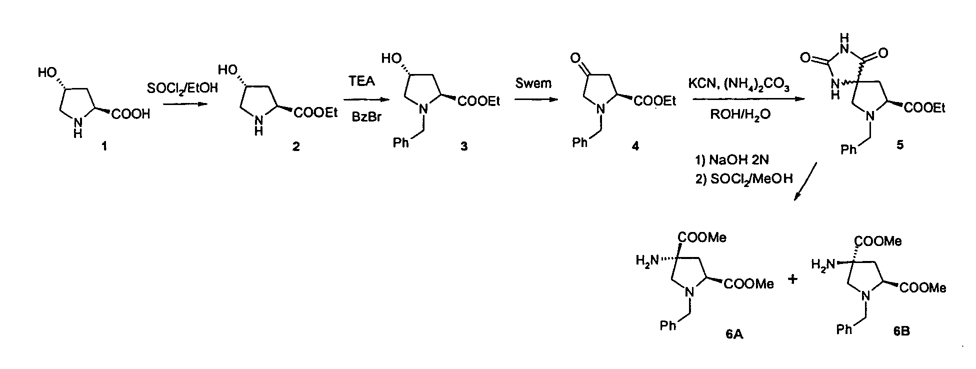 The preferred starting material is (2S,4R)-4-hydroxyproline (compound **1**), a current commercial product (Sigma, Acros, Lancaster). Through a series of reactions, this material is converted into a carboxi- and amino-protected analog **3** which is oxidized to obtain the compound **4.** This material is then converted in its hydantoin analog **5.** This last step results in the formation of diastereoisomers which are not separated at this stage of the synthesis. Next step involves the transformation of the hydantoin moiety into the corresponding alpha-aminoester group (compound **6**). The two diastereoisomers **6A** and 6B are separated using standard chromatography procedure.
The cis diastereoisomer **6A** is then successively protected into its boc analog **7A** and debenzylated to afford the secondary amine **8A.** The latter compound is used as a platform for the reaction with different electrophylic species such as acid chlorides (see scheme below). Product **9A** obtained is finally deprotected to give the expected derivative **10A** corresponding of formula (II): 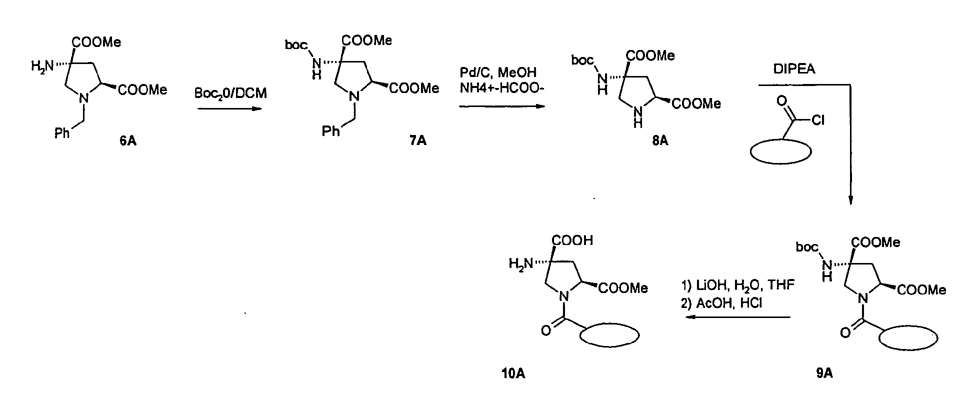 Examples of electrophylic species: 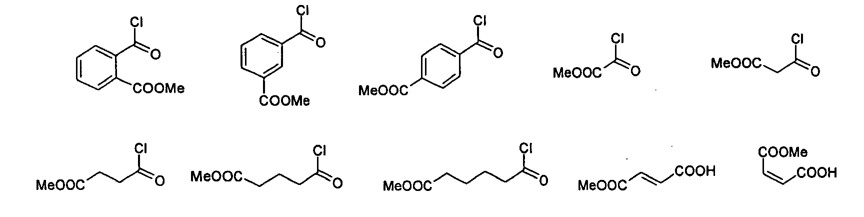 used to obtain the following compounds: 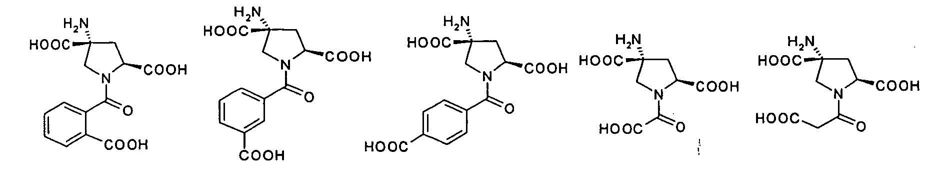 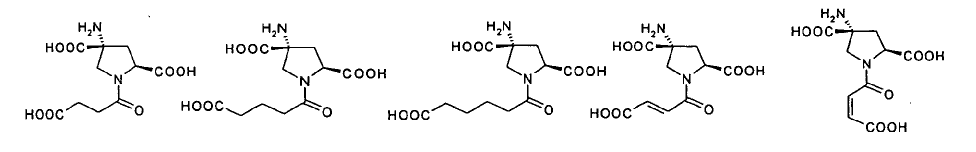 Other compounds according to general formula (I) can easily be obtained by one skilled in the art from the commercial starting material (2R,4R)-4-hydroxyproline (Acros, Sigma) using the same synthetic routes described above for compounds corresponding to formula (II).

Within the meaning of the present invention, the term "compound" means a compound of the general formula (I) or a compound of the general formula (II) but also a mixed preparation of compounds of formula (I) and its corresponding enantiomer of formula (II). It can also be a mixed preparation of compounds of formula (I) plus one of its (2,4-COOH)-trans-diastereoisomers or a compound of formula (II) plus one of its (2,4-COOH)-trans-diastereoisomers. Consequently the term "compound of formulae (I) and/or (II)" refers to a compound of formula (I), a compound of formula (II), a compound of formula (I) plus its corresponding enantiomer of formula (II) in mixture, a compound of formula (I) mixed with one of its (2,4-COOH)-trans-diastereoisomer, a compound of formula (II) mixed with one of its (2,4-COOH)-trans-diastereoisomer or enantiomers of formulae (I) and (II) mixed with at least one of their corresponding (2,4-COOH)-trans-diastereoisomers.

As used herein, the term "nervous system" refers to Central Nervous System (CNS) plus Peripheral Nervous System (PNS).

This invention relates to new *cis* pyrrolidinyl derivatives as defined above, pharmaceutical compositions containing them and their use for the treatment and/or prophylaxis of conditions associated with altered glutamatergic signalling and/or functions, and/or conditions which can be affected by alteration of glutamate level or signalling in mammals.

According to yet another aspect, the present invention provides pharmaceutical compositions which comprise a compound of the general formula (I), (Ia), (Ib), (II), (IIa) and/or (IIb), as defined above, or a pharmaceutically acceptable salt or a metabolically labile ester or amide thereof, in combination with a pharmaceutical acceptable carrier.

The invention relates more particularly to pharmaceutical compositions comprising a pharmaceutically acceptable carrier and a pharmaceutically effective amount of a compound of general formula (I), (Ia), (Ib), (II), (IIa) and/or (IIb), as defined above, in association with their corresponding (2,4)-COOR₁/R₂ trans-diastereoisomers mentioned above, a pharmaceutically acceptable salt or a metabolically labile ester or amide thereof.

The pharmaceutically acceptable salts of the formulae (I), (Ia), (Ib), (II), (IIa) and/or (IIb), as defined above, can exist in conjunction with the acidic or basic portion of the compound and can exist as acid addition, primary, secondary, tertiary, or quaternary ammonium, alkali metal, or alkaline earth metal salts. Generally, the acid addition salts are prepared by the reaction of an acid with a compound of formula (I), (Ia), (Ib), (II), (IIa) and/or (IIb), as defined above. The alkali metal and alkaline earth metal salts are generally prepared by the reaction of the hydroxide form of the desired metal salt with a compound of formula (I), (Ia), (Ib), (II), (IIa) and/or (IIb), as defined above. Examples of pharmaceutically acceptable acid addition salts include those derived from inorganic acids such as hydrochloric, hydrobromic, nitric, carbonic, formic, monohydrogencarbonic, phosphoric, monohydrogenphosphoric, dihydrogenphosphoric, perchloric, sulfuric, monohydrogensulfuric, hydriodic, or phosphorous acids and the like, as well as the salts derived from organic acids like acetic, lactic, propionic, butyric, isobutyric, palmoic, maleic, glutamic, hydroxymaleic, malonic, benzoic, succinic, glycolic, suberic, fumaric, mandelic, phthalic, salicylic, benzenesulfonic, p-tolylsulfonic, citric, tartaric, methanesulfonic, hydroxynaphthoic, hydroiodic, and the like. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, lithium, calcium, aluminium, ammonium, barium, zinc, organic amino, or magnesium salt, N,N¹-dibenzylethylenediamine, choline, diethanolamine, ethylenediamine, N-methylglucamine, procaine salts (e.g. chloroprocaine) and the like. Also included are salts of amino acids such as arginate and the like, and salts of organic acids like glucuronic or galacturonic acids and the like (see, for example, Berge et al, "Pharmaceutical Salts", Journal of Pharmaceutical Science, 66, 1-19). Finally, certain specific compounds of the present invention contain both basic and acidic functionalities that allow the compounds to be converted into either base or acid addition salts.

The pharmaceutically acceptable metabolically labile ester and amide of compounds of formulae (I), (Ia), (Ib), (II), (IIa) and/or (IIb), as defined above, are ester or amide derivatives of compounds of formula (I), (Ia), (Ib), (II), (IIa) and/or (IIb), as defined above, that are hydrolized *in vivo* to afford said compound of formula (I), (Ia), (Ib), (II), (IIa) and/or (IIb), as defined above, and a pharmaceutically acceptable alcohol or amine. Examples of metabolically labile esters include esters formed with (1-6C) alkanols in which the alkanol moiety may be optionally substituted by a (1-8C) alkoxy group, for example methanol, ethanol, propanol and methoxyethanol. Examples of metabolically labile amides include amides formed with natural or non-natural amino acids.

In a further aspect, the present invention provides pharmaceutical compositions which comprise a compound of the general formula (I), (Ia), (Ib), (II), (IIa) and/or (IIb), as defined above, a pharmaceutically acceptable salt or a metabolically labile ester or amide thereof, in combination with a pharmaceutical acceptable carrier, diluent or excipient. The pharmaceutical compositions are prepared by known procedures using well-known and readily available ingredients. In making the compositions of the present invention, the active ingredient will usually be mixed with a carrier, or diluted by a carrier, or enclosed with a carrier, and may be in the form of a capsule, sachet, paper, or other container. When the carrier serves as a diluent, it may be a solid, semi-solid, or liquid material which acts as a vehicle, excipient, or medium for the active ingredient. The compositions can be in the form of tablets, pills, powders, lozenges, sachets, cachets, elixirs, suspensions, emulsions, solutions, syrups, aerosols, ointments containing, for example up to 10% by weight of active compound, soft and hard gelatin capsules, suppositories, sterile injectable solutions, and sterile packaged powders.

Some examples of suitable carriers, excipients and diluents include lactose, dextrose, sucrose, sorbitol, mannitol, starches, gum, acacia, calcium phosphate, alginates, gelatin, calcium silicate, microcrystalline cellulose, water syrup, methyl cellulose, methyl and propyl hydrobenzoates, talc, magnesium stearate and mineral oil. In addition, the compositions can include lubricating agents, wetting agents, preservatives, solubilizers, stabilizers, emulsifiers, sweeteners, colorants, flavorants, salts for varying the osmotic pressure, buffers, masking agents and antioxidants. They can also contain still other therapeutically valuable substances. The compositions of the invention may be formulated so as to provide quick, sustained, or delayed release of the active ingredient after administration to the patient by employing procedures well known in the art.

The invention relates more particularly to compounds (I), (Ia), (Ib), (II), (IIa) and/or (IIb), as defined above, consisting of modulators of nervous system receptors sensitive to glutamate.

More particularly the nervous system receptors sensitive to glutamate, are metabotropic glutamate receptors.

In preferred embodiments, the compounds of formulae (I), (Ia), (Ib), (II), (IIa) and/or (IIb), as defined above, are modulators of metabotropic glutamate receptors (mGluRs) functions, and are especially agonists, antagonists or reverse agonists of mGluRs functions.

The ability of compounds of general formula (I) and/or (II) to modulate mGluRs functions may be demonstrated by examining their ability to influence inositol phosphates (IP) production and accumulation (Gomeza et al., Molecular Pharmacology, 1996, 50:923-930) in transfected cells expressing individual mGluRs subtypes coupled to particular G proteins. Using that method, if the compound is an agonist of the mGluR subtype, the IP production in cells will be significantly (p<0,05) above the basal IP formation. To assess the antagonist activity of a compound of the invention, a comparison shall be performed between the IP production induced by Glu EC₈₀ alone and the combination of the compound plus Glu EC₈₀; if it is significantly lower than the Glu EC₈₀ IP formation we are in presence of an antagonist of the mGluR subtype. Finally, if the compound of the invention is a reverse agonist of the mGluR subtype, the measured IP production will be significantly decreased compared to the basal IP formation. If the compound of the invention is an allosteric modulator of the mGluR subtype function, a comparison shall be performed between the IP production induced by Glu EC₃₀ alone and the combination of the compound plus Glu EC₃₀; if it is significantly higher than the Glu EC₃₀ IP formation we are in presence of an allosteric modulator of the mGluR subtype.

In a particular embodiment, the compounds of formulae (I), (Ia), (Ib), (II), (IIa) and/or (IIb), as defined above, are modulators of mGluRs of group III functions, and are especially agonists, antagonists or reverse agonists of these mGluR group III functions; members of group III being mGluR4, mGluR6, mGluR7 and mGluR8. Using the test directed to the ability of the compounds to influence IP production and accumulation described above, an activity shall only be observed when the transfected cells are expressing mGluRs of group III.

In another aspect, the compounds of formulae (I), (Ia), (Ib), (II), (IIa) and/or (IIb), as defined above, are subtype selective modulators of mGluR group III functions, and are especially agonists, antagonists or reverse agonists of these mGluRs group III functions; the members of group III being mGluR4, mGluR6, mGluR7 and mGluR8. Using the test directed to the ability of the compounds to influence IP production and accumulation described above, an activity shall only be observed when the transfected cells are expressing one of the mGluRs of group III subtypes; these subtypes being mGluR4, mGluR6, mGluR7 and mGluR8.

According to a preferred embodiment, the compounds of formulae (I), (Ia), (Ib), (II), (IIa) and/or (IIb), as defined above, are subtype selective modulators of mGluRs of group III functions, and are especially agonists, antagonists or reverse agonists of mGluR4, mGluR7 and mGluR8 which are the mGluRs of group III found in the central nervous system. Using the test directed to the ability of the compounds to influence IP production and accumulation described above, an activity shall only be observed when the transfected cells are expressing one of the central nervous system mGluRs of group III subtypes; these subtypes being mGluR4, mGluR7 and mGluR8.

In a preferred aspect, the compounds of formulae (I), (Ia), (Ib), (II), (IIa) and/or (IIb), as defined above, are subtype selective agonists of mGluRs of group III functions.

According to a particular embodiment, the compounds of formulae (I), (Ia), (Ib), (II), (IIa) and/or (IIb), as defined above, are subtype selective agonists of mGluR4, mGluR7 or mGluR8.

In another aspect, the present invention provides a method of modulating mGluRs functions in a mammal including a human, which comprises administering an effective amount of a compound of formula (I), (Ia), (Ib), (II), (IIa) and/or (IIb), as defined above, or a pharmaceutically acceptable salt, or a metabolically labile ester or amide thereof, to a patient in need thereof. It further relates to a method of modulating mGluRs of group III functions by administering an effective amount of a compound of formula (I), (Ia), (Ib), (II), (IIa) and/or (IIb), or a pharmaceutically acceptable salt, or a metabolically labile ester or amide thereof, to a patient in need thereof.

The invention also concerns a method of modulating metabotropic glutamate receptors functions in a mammal, including a human as mentioned above, which comprises administering an effective amount of a compound of formula (I), (Ia), (Ib), (II), (IIa) and/or (IIb), as defined above, optionally in association with their corresponding (2,4)-COOR₁/R₂ trans-diastereoisomers mentioned above, or pharmaceutically acceptable salt, or metabolically labile ester or amide thereof.

In a particular aspect, the present invention provides a method of modulating glutamate signalling in a mammal including a human, which comprises administering an effective amount of a compound of formula (I) or (II), or a pharmaceutically acceptable salt, or a metabolically labile ester or amide thereof, to a patient in need thereof.

According to another aspect, the present invention provides a method of modulating glutamate levels in a mammal including a human, which comprises administering an effective amount of a compound of formula (I) or (II), or a pharmaceutically acceptable salt, or a metabolically labile ester or amide thereof, to a patient in need thereof.

The particular dose of compound of general formula (I), (Ia), (Ib), (II), (IIa) and/or (IIb), as defined above, administered according to this invention shall be determined by the particular circumstances surrounding the case, including the compound administered, the route of administration, the particular condition being treated, and similar considerations. The compounds can be administered by a variety of routes including oral, rectal, transdermal, subcutaneous, intravenous, intramuscular, or intranasal routes. Additionally, the compound of the invention may be administered by continuous infusion. A typical daily dose shall contain from about 0,01 mg/kg to about 100 mg/kg of the active compound of the invention. Preferably, daily doses will be about 0,05 mg/kg to about 50 mg/kg, more preferably from about 0,1 mg/kg to about 25 mg/kg.

The invention also relates to the use of a compound of general formula (I), (Ia), (Ib), (II), (IIa) and/or (IIb), as defined above (which can be in association with their corresponding (2,4)-COOR₁/R₂ trans-diastereoisomers mentioned above), for the manufacture of a drug for the treatment and/or prophylaxis of a condition associated with altered glutamatergic signalling and/or functions, and/or conditions which can be affected by alteration of glutamate level or signalling.

Such conditions associated with altered glutamatergic signalling and/or functions, and/or conditions which can be affected by alteration of glutamate level or signalling as detailed above are selected from, but not limited to epilepsy, dementias (including dementias of the Alzheimer's type, vascular dementias, AIDS-dementia complex, etc...), parkinsonism and movement disorders (including Huntington's disease, dystonias, Gilles de la Tourette syndrome, dyskinesias etc...), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis, etc...), headache, migraine, myoneural disorders (myasthenia gravis, acute muscle spasms, myopathies, etc...), disorders of the eye andvisual pathways, intracranial trauma/injury, trauma/injury to nerves and spinal cord, poisoning and toxic effects of nonmedicinal substances, accidental poisoning by drugs medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including, attention deficit and disruptive behaviour disorders, autism, TIC disorders, etc ...), delirium and other cognitive disorders, substance related disorders (alcohol, nicotine, drugs), schizophrenia and other psychotic disorders, mood disorders (including depressive disorders and bipolar disorders), anxiety disorders, sexual disorders, eating disorders, sleep disorders, endocrine and metabolic diseases (diabetes, hypoglycaemia), acute and chronic pain; nausea and vomiting; irritable bowel syndrome.

According to the invention, the terms "treatment and/or prophylaxis" refer to a process that is intended to produce a beneficial change in the condition of a mammal, e.g., a human, often referred to as a patient. A beneficial change can, for example, include one or more of: restoration of function, reduction of symptoms, limitation or retardation of progression of a disease, disorder, or condition or prevention, limitation or retardation of deterioration of a patient's condition, disease or disorder, improvement of the patient's quality of life. Such therapy can involve, for example, nutritional modifications, administration of radiation, administration of a drug, behavioral modifications, and combinations of these, among others.

All publications and patent applications cited in this specification are herein incorporated by reference as if each individual publication or patent application were specifically and individually indicated to be incorporated by reference. Although the foregoing invention has been described in some detail by way of illustration and example for purposes of clarity of understanding, it will be readily apparent to those of ordinary skill in light of the teachings of this invention that certain changes and modifications may be made thereto without departing from the spirit or scope of the appended claims.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practised otherwise than as specifically described. Accordingly, those skilled in the art will recognize, or able to ascertain using no more than routine experimentation, many equivalents to the specific embodiments of the invention described specifically herein. Such equivalents are intended to be encompassed in the scope of the following claims.

## Claims

1. A compound of the formula (I) or (II) 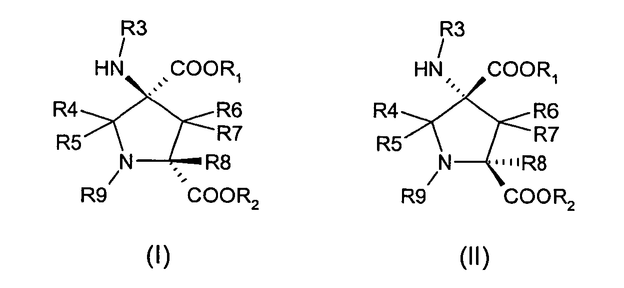 in which:
**R**_{**1**} and **R**_{**2**} are each individually hydrogen or a carboxy-protecting group;
**R**_{**3**} is hydrogen or an amino-protecting group;
**R**_{**4**} to **R**_{**8**}**,** identical to or different from each other, represent a hydrogen atom, a halogen atom, an alkyl radical or an aryl radical, a -OH or a -SH, these radicals themselves being substituted where appropriate;
**R**_{**4**} and **R**_{**5**} can form a carbonyl bond or a thiocarbonyl bond;
**R**_{**9**} represents a **(R**_{**10**}**)**_{**n**}**(-R**_{**11**}**)**_{**m**} group wherein
**n** represents an integrer of from 0 to 4;
**m** represents an integrer of from 1 to 3;
**R**_{**10**} is a moiety selected in the group consisting of:
(i) CH₂
(ii) 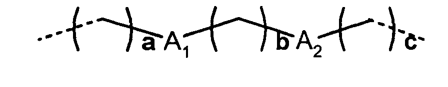
(iii) 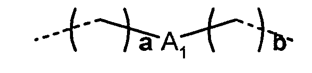 with:
**a, b** and **c** are, independently from one another, an integer ranging from 0 to 4 **;**
**A**_{**1**} and **A**_{**2**} are, independently from one another, a moiety selected in the group consisting of -CO-, -CS-, -0-, -S-, -SO-, -SO₂-, -COO-, -CON(C_{n'}H_{2n'+1})-, -N(C_{n'}H_{2n'+1})CO-, -CSN(C_{n'}H_{2n'+1})-, -N(C_{n'H2n'+1})CS-, -N(C_{n'}H_{2n'+1})-, -(C_{n'}H_{2n'+1})-, aryl, cycloalkyl, -1,4-piperidinyl, 1,4-piperazinyl,
with **-(C**_{**n**}**,H**_{**2n'+1**}**)-** designating a straight or branched chain, or cyclic carbon radical, or combination thereof, which may be fully saturated, mono or polyunsaturated and can include di- and multi-moieties, and having from 1 to 8 carbon atoms,
with n' is, independently from one another, an integer ranging from 0 to 8, preferably from 1 to 4, preferably from 1 to 3 and more preferably from 1 to 2,
**R**_{**11**} is a polar group such as -COOH, -SO₃H, -SO₂H, -PO₃H₂, -PO₂H, -B(OH)₂, tetrazol, -CO (C_{m'}H_{2m'+1}), -C (NOH)(C_{m'}H_{2m'+1}), -CS (C_{m'}H_{2m'+1}), -OH, -O(C_{m'}H_{2m'+1}), -OCO(C_{m'}H_{2m'+1}), -SH, -S(C_{m'}H_{2m'+1}), -SCO(C_{m'}H_{2m'+1}), -NH₂, -NHOH, -N(C_{m'}H_{2m'+1})₂, -N⁺(C_{m'}H_{2m'+1})₃, -NHCO (C_{m'}H_{2m'+1}), -NHSO₂(C_{m'}H_{2m'+1})₂, -NHCON (C_{m'}H_{2m'+1}), -NHCSN(C_{m'}H_{2m'+1}), -CON(C_{m'}H_{2m'+1})₂, -CSN(C_{m'}H_{2m'+1})₂, -SO₂N(C_{m'}H_{2m'+1})₂;
with **m'** is, independently from one another, an integer ranging from 0 to 4,
as well as their pharmaceutically acceptable salts, or their metabolically labile esters or amides.

2. Compounds according to claim 1, of the formula (Ia) or (IIa) 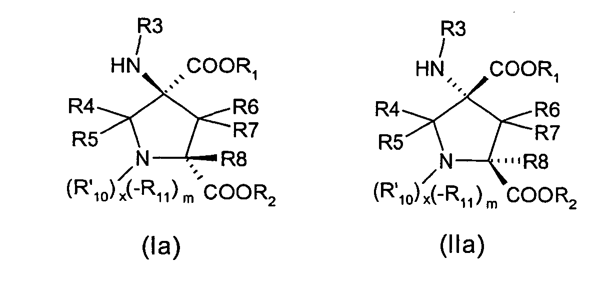 in which:
**R**_{**1**} to **R**_{**8**}**, R**_{**11**}**,** and **m** are as defined in claim 1,
**X** represents an integer from 1 to 4;
**R'**_{**10**} is a moiety selected in the group consisting of:
(i) 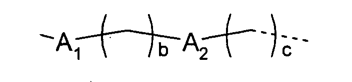
(ii) 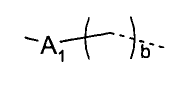 with:
**b**, **c**, **A**_{**1**} and **A**_{**2**} being as defined in claim 1.

3. Compounds according to claim 1 or 2, of the formula (Ia) or (IIa) wherein **A**_{**1**} is a moiety selected in the group consisting of -CO-, -CS-, or -SO₂-.

4. Compounds according to any of claims 1 to 3, of the formula (Ia) or (IIa) wherein **A**_{**2**} is a moiety selected in the group consisting of aryl, -NH-, or -(C_{n'}H_{2n'+1})- as defined in claim 1.

5. Compounds according to any one of claims 1 to 4, of the formula (Ib) or (IIb) 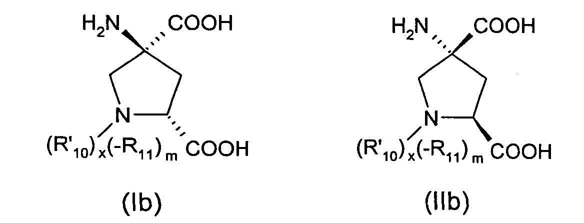 in which **R'**_{**10**}**, R**_{**11**}**, x** and **m** are as defined in claims 2 to 4.

6. Compounds according to any one of claims 1 to 5, of the formula (I), (Ia), (Ib),(II), (IIa), or (IIb) wherein **R**_{**11**} is an acidic group such as -COOH, -SO₃H, -SO₂H, -PO₃H₂, -PO₂H, -B(OH)₂, and tetrazol.

7. Compounds according to any one of claims 1 to 6, corresponding to:
(2S,4R)-4-Amino-pyrrolidine-1,2,4-tricarboxylic acid ;
(2R,4S)-4-Amino-pyrrolidine-1,2,4-tricarboxylic acid ;
(2S,4R)-4-Amino-1-oxalyl-pyrrolidine-2,4-dicarboxylic acid;
(2R,4S)-4-Amino-1-oxalyl-pyrrolidine-2,4-dicarboxylic acid;
(2S,4R)-4-Amino-1-(2-carboxy-acetyl)-pyrrolidine-2,4-dicarboxylic acid;
(2R,4S)-4-Amino-1-(2-carboxy-acetyl)-pyrrolidine-2,4-dicarboxylic acid;
(2S,4R)-4-Amino-1-(3-carboxy-propionyl)-pyrrolidine-2,4-dicarboxylic acid;
(2R,4S)-4-Amino-1-(3-carboxy-propionyl)-pyrrolidine-2,4-dicarboxylic acid;
(2S,4R)-4-Amino-1-(4-carboxy-butyryl)-pyrrolidine-2,4-dicarboxylic acid;
(2R,4S)-4-Amino-1-(4-carboxy-butyryl)-pyrrolidine-2,4-dicarboxylic acid;
(2S,4R)-4-Amino-1-(5-carboxy-pentanoyl)-pyrrolidine-2,4-dicarboxylic acid;
(2R,4S)-4-Amino-1-(5-carboxy-pentanoyl)-pyrrolidine-2,4-dicarboxylic acid;
(2S,4R)-4-Amino-1-((E)-3-carboxy-acryloyl)-pyrrolidine-2,4-dicarboxylic acid;
(2R,4S)-4-Amino-1-((E)-3-carboxy-acryloyl)-pyrrolidine-2,4-dicarboxylic acid;
(2S,4R)-4-Amino-1-((Z)-3-carboxy-acryloyl)-pyrrolidine-2,4-dicarboxylic acid;
(2R,4S)-4-Amino-1-((Z)-3-carboxy-acryloyl) -pyrrolidine-2,4-dicarboxylic acid;
(2S,4R)-4-Amino-1-(2-carboxy-benzoyl)-pyrrolidine-2,4-dicarboxylic acid;
(2R,4S)-4-Amino-1-(2-carboxy-benzoyl)-pyrrolidine-2,4-dicarboxylic acid;
(2S,4R)-4-Amino-1-(3-carboxy-benzoyl)-pyrrolidine-2,4-dicarboxylic acid;
(2R,4S)-4-Amino-1-(3-carboxy-benzoyl)-pyrrolidine-2,4-dicarboxylic acid;
(2S,4R)-4-Amino-1-(4-carboxy-benzoyl)-pyrrolidine-2,4-dicarboxylic acid;
(2R,4S)-4-Amino-1-(4-carboxy-benzoyl)-pyrrolidine-2,4-dicarboxylic acid;
(2S,4R)-4-Amino-1-(2-carboxy-benzenesulfonyl)-pyrrolidine-2,4-dicarboxylic acid;
(2R,4S)-4-Amino-1-(2-carboxy-benzenesulfonyl)-pyrrolidine-2,4-dicarboxylic acid;
(2S,4R)-4-Amino-1-(3-carboxy-benzenesulfonyl)-pyrrolidine-2,4-dicarboxylic acid;
(2R,4S)-4-Amino-1-(3-carboxy-benzenesulfonyl)-pyrrolidine-2,4-dicarboxylic acid;
(2S,4R)-4-Amino-1-(4-carboxy-benzenesulfonyl)-pyrrolidine-2,4-dicarboxylic acid;
(2R,4S)-4-Amino-1-(4-carboxy-benzenesulfonyl) -pyrrolidine-2,4-dicarboxylic acid;
(2S,4R)-4-Amino-1-(3-carboxy-propylcarbamoyl)-pyrrolidine-2,4-dicarboxylic acid;
(2R,4S)-4-Amino-1-(3-carboxy-propylcarbamoyl)-pyrrolidine-2,4-dicarboxylic acid;
(2S,4R)-4-Amino-1-(3-carboxy-propylthiocarbamoyl)-pyrrolidine-2,4-dicarboxylic acid;
(2R,4S)-4-Amino-1-(3-carboxy-propylthiocarbamoyl)-pyrrolidine-2,4-dicarboxylic acid.

8. Compounds according to any one of claims 1 to 5, of the formula (I), (Ia), (Ib),(II), (IIa), or (IIb) wherein **R**_{**11**} is a group selected from -CO (C_{m'}H_{2m'+1}), -C (NOH)(C_{m'}H_{2m'+1}), -CS (C_{m'}H_{2m'+1}), -OH, -O(C_{m'}H_{2m'+1}), -OCO (C_{m'}H_{2m'+1}), -SH, -S (C_{m'}H_{2m'+1}), -SCO (C_{m'}H_{2m'+1}), -NH₂, -NHOH, -N (C_{m'}H_{2m'+1})₂, -N⁺(C_{m'}H_{2m'+1})₃, -NHCO (C_{m'}H_{2m'+1}), -NHSO₂(C_{m'}H_{2m'+1})₂, -NHCON (C_{m'}H_{2m'+1}), -NHCSN (C_{m'}H_{2m'+1}), -CON (C_{m'}H_{2m'+1})₂, -CSN (C_{m'}H_{2m'+1})₂, -SO₂N(C_{m'}H_{2m'+1})₂; with m' being as defined in claim 1.

9. Compounds according to any one of claims 1 to 5 and 8, corresponding to :
(2S,4R)-4-Amino-1-(2-hydroxy-acetyl)-pyrrolidine-2,4-dicarboxylic acid;
(2R,4S)-4-Amino-1-(2-hydroxy-acetyl)-pyrrolidine-2,4-dicarboxylic acid;
(2S,4R)-4-Amino-1-(2-methoxy-acetyl)-pyrrolidine-2,4-dicarboxylic acid;
(2R,4S)-4-Amino-1-(2-methoxy-acetyl)-pyrrolidine-2,4-dicarboxylic acid;
(2S,4R)-1-(2-Acetylamino-acetyl)-pyrrolidine-2,4-dicarboxylic acid;
(2R,4S)-1-(2-Acetylamino-acetyl)-pyrrolidine-2,4-dicarboxylic acid.

10. Compounds according to any one of claims 1 to 9, consisting of modulators of nervous system receptors sensitive to glutamate.

11. Compounds according to claim 10, where the nervous system receptors sensitive to amino acids are metabotropic glutamate receptors.

12. Compounds according to claim 10, being in particular agonists, antagonists or reverse agonists of the metabotropic glutamate receptors functions.

13. A pharmaceutical composition comprising a pharmaceutically acceptable carrier and a pharmaceutically effective amount of a compound of general formula (I) and/or (II) according to any one of claims 1 to 12, a pharmaceutically acceptable salt or a metabolically labile ester or amide thereof.

14. A pharmaceutical composition comprising a pharmaceutically acceptable carrier and a pharmaceutically effective amount of a compound of general formula (I) and/or (II) according to any one of claims 1 to 12, in association with their corresponding (2,4)-COOR₁/R₂ trans-diastereoisomers, a pharmaceutically acceptable salt or a metabolically labile ester or amide thereof.

15. Use of a compound of general formula (I) and/or (II) according to any one of claims 1 to 12, for the manufacture of a medicament for the treatment and/or prophylaxis of a condition associated with altered glutamatergic signalling and/or functions, and/or conditions which can be affected by alteration of glutamate level or signalling.

16. Use according to claim 15, of a compound of general formula (I) and/or (II) according to any one of claims 1 to 12, in association with their corresponding (2,4)-COOR₁/R₂ trans-diastereoisomers, for the manufacture of a medicament for the treatment and/or prophylaxis of a condition associated with conditions associated with altered glutamatergic signalling and/or functions, and/or conditions which can be affected by alteration of glutamate level or signalling.

17. Use according to claim 15 or 16, where the condition associated with altered glutamatergic signalling and/or functions, and/or conditions which can be affected by alteration of glutamate level or signalling is selected from epilepsy, dementias (including dementias of the Alzheimer's type, vascular dementias, AIDS-dementia complex, etc...), parkinsonism and movement disorders (including Huntington's disease, dystonias, Gilles de la Tourette syndrome, dyskinesias etc...), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis, etc...), headache, migraine, myoneural disorders (myasthenia gravis, acute muscle spasms, myopathies, etc...), disorders of the eye andvisual pathways, intracranial trauma/injury, trauma/injury to nerves and spinal cord, poisoning and toxic effects of nonmedicinal substances, accidental poisoning by drugs medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including, attention deficit and disruptive behaviour disorders, autism, TIC disorders, etc ...), delirium and other cognitive disorders, substance related disorders (alcohol, nicotine, drugs), schizophrenia and other psychotic disorders, mood disorders (including depressive disorders and bipolar disorders), anxiety disorders, sexual disorders, eating disorders, sleep disorders, endocrine and metabolic diseases (diabetes, hypoglycaemia), acute and chronic pain; nausea and vomiting; irritable bowel syndrome.
